# EUROPEAN PATENT APPLICATION

(11) **EP 4 309 652 A1**
(43) Date of publication of application: **24.01.2024**
(21) Application number: 22382696.7
(22) Date of filing: 20.07.2022
(51) Int. Cl.: A61K 31/351, A61P 19/02, A61P 29/00

(54) **COMPOSITION FOR USE IN THE PREVENTION AND/OR TREATMENT OF DISEASES MEDIATED BY TLR4, IL1R, COX1/2 AND/OR RBP4**

(71) Applicant: Servizo Galego de Saúde (SERGAS), 15703 Santiago de Compostela (ES); Fundación Instituto de Investigación Sanitaria de Santiago de Compostela, 15706 Santiago de Compostela, A Coruña (ES); Universidade Federal De Sergipe, São Cristovão, Sergipe (BR)
(72) Inventor: GOMEZ BAHAMONDE, Rodolfo, 15706 Santiago de Compostela, A Coruña (ES); ALMADA DA SILVA, James, São Cristóvão, Sergipe (BR); LÓPEZ FAGÚNDEZ, Miriam, 15706 Santiago de Compostela, A Coruña (ES); FRANCO TREPAT, Eloi, 15706 Santiago de Compostela, A Coruña (ES); ALONSO PÉREZ, Ana, 15706 Santiago de Compostela, A Coruña (ES); GUILLÁN FRESCO, María, 15706 Santiago de Compostela, A Coruña (ES); PAZOS PÉREZ, Andrés, 15706 Santiago de Compostela, A Coruña (ES); JORGE MORA, Alberto Agustín, 15706 Santiago de Compostela, A Coruña (ES)
(74) Representative: Hoffmann Eitle

(57) **Abstract**

The present invention refers to a composition for use in the prevention and/or treatment of diseases mediated by TLR4, IL1R, COX1/2 and/or RBP4, preferably inflammatory diseases such as Osteoarthritis (OA) or other diseases, such as infectious diseases, causing an inflammatory response.

## Description

### FIELD OF THE INVENTION

The present invention refers to the medical field. Particularly, the present invention refers to a composition for use in the prevention and/or treatment of diseases mediated by TLR4, IL1R, COX1/2 and/or RBP4, preferably inflammatory diseases such as, for example, Osteoarthritis (OA) or other diseases, such as infectious diseases, causing an inflammatory response.

### STATE OF THE ART

OA is the most common chronic rheumatic disease. It is mainly characterized by joint space narrowing due to progressive degradation of the articular cartilage. These joint alterations are associated to pain, disability, and loss of joint architecture. Cartilage is the main tissue altered during OA progression. However, it also affects other joint tissues including bone, synovium, and tendons. Accordingly, it is considered a whole joint pathology. It is well-known that biomechanical alterations are the driving force in this pathology. Interestingly, it has been widely described that inflammation contributes to its onset and progression. This inflammation is frequently treated using cyclooxygenase enzyme (COX) inhibitors. Nonetheless, this pharmacological approach does not block OA-induced inflammation because OA inflammation is also sustained by innate immune response mediated innate immune receptors like TLR4 and IL1R.

It was demonstrated that COX enzymes have an active role in joint degenerative diseases. This enzyme regulates the formation of important biological mediators called prostanoids, including prostaglandins, prostacyclin's, and thromboxane's. In fact, prostaglandin (PG) causes pain during inflammation. An inflammatory environment induces COX-2 production and stimulates angiogenesis, supporting OA catabolic and inflammatory processes. According to COX properties, it was extensively used as a therapeutic target to treat inflammation and pain in OA and rheumatoid arthritis (RA) patients. However, it is well-known that COX inhibitors do not block the origin of OA inflammation and only help to control the symptoms, especially pain.

IL-1β belongs to a family of well-known pro-catabolic and inflammatory factors. It is an inducer of cartilage degradation but also drives synovitis. Accordingly, it has been demonstrated that IL-1β expression was higher in OA patients. Nonetheless, IL-1 blockade alone did not improve OA progression. However, IL-1 has proven to be a good target in other inflammatory rheumatic diseases like rheumatoid arthritis and gout, among others.

The receptors from the toll-like receptor family (TLRs) are also linked to the development of articular IIRs. TLRs can recognize pathogen-associated molecular patterns (PAMPs) but they also recognize damage-associated molecular patterns (DAMPS), which are host-derived molecules released by different cell types when tissues are damaged. These DAMPS and PAMPS are highly involved in the development of OA and other rheumatic diseases. TLRs, like TLR4, have been deeply involved in articular inflammatory responses in the OA context. However, currently, there is no approved inhibitor to block their activation.

Specifically, TLR4- and IL1R-mediated responses are associated with cytokine production like lipocalin-2 (LCN2) and interleukin-6 (IL-6), as well as to other catabolic and inflammatory-related factors such as nitric oxide synthase (NOS₂), cyclooxygenases enzymes (COX ½) and metalloproteinases (MMPs).

Multiple rheumatic diseases, including OA, are tightly linked with metabolic alterations that significantly contribute to their onset and progression. Among the factors supporting this relationship are adipokines, like RBP4. This adipokine has been associated with the development of several metabolic disorders such as diabetes, obesity, or metabolic syndrome. In addition, it also has a relevant role in other diseases characterised by a chronic inflammatory state like arthritis. Consistent with this, previous reports have revealed that RBP4 may bind to and activate Toll-like receptor 4 (TLR4). This means that RBP4 is considered a damage-associated molecular pattern (DAMP), which are factors released in pathologic circumstances where tissues are being damaged and participate in the inflammatory responses. Nonetheless, currently, there is no available therapy to reduce RBP4 activity.

Consequently, there is an unmet medical need of identifying new candidates to be used in the prevention and/or treatment of diseases mediated by TLR4, IL1R, COX1/2 and/or RBP4, preferably inflammatory diseases, such as OA, which, at the same time, do not show toxic effects.

The present invention is focused on solving this technical problem and new compounds for said intended medical use are herein provided.

### DESCRIPTION OF THE INVENTION

### Brief description of the invention

The present invention refers to a composition for use in the prevention and/or treatment of diseases mediated by TLR4, IL1R, COX1/2 and/or RBP4, preferably inflammatory diseases such as OA or other diseases, such as infectious diseases, causing an inflammatory response.

Particularly, three structurally related active compounds have been isolated and assayed in the present invention. Such as it is shown in the results provided in the present invention, *in silico* and *in vitro* experiments were performed in chondrocytes and osteoblasts and the RT-PCR, biochemical assays, MTT assays, and western blot results revealed that these compounds exhibit anti-TLR4 (**Example 2.2, 2.5 and 2.8**), anti-IL1R (**Example 2.2, 2.6 and 2.8**), and anti-COX1/2 (**Example 2.2 and 2.7**) activities without showing any toxic effect. Moreover, these compounds are able to reduce the expression of RBP4 (**Examples 2.9**).

Consequently, according to the data presented in the present invention, these compounds, through their anti-TLR4, anti-IL1R, and anti-COX1/2 activities, and the reduction of RBP4, might be a potential pharmacological tool in the prevention and/or treatment of diseases mediated by TLR4, IL1R, COX1/2 and/or RBP4, preferably inflammatory diseases such as OA.

So, the first embodiment of the present invention refers to a compound of Formula (I) (herein after *compound of the invention),* or salts derived thereof, wherein **OAc** is an acetyloxy group and **R** may be substituted by **OH**, **H** or **OAc**, 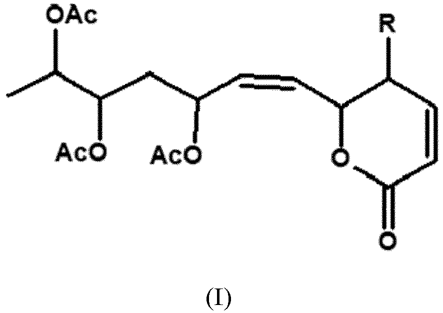 for use in the prevention and/or treatment of diseases mediated by TLR4, IL1R, COX1/2 and/or RBP4.

Kindly note that the compounds of the invention are also identified in the present invention as follows:
- Pectinolide J (R = OH).
- Hyptolide (R = H).
- Pectinolide (R = OAc).

On the other hand, please note that the diseases which may be mediated by TLR4, IL1R, COX1/2 and/or RBP4 pertain to the common general knowledge. In other words, the person skilled in the art, using the common general knowledge, would undoubtedly recognize the diseases which are mediated by TLR4, IL1R, COX1/2 and/or RBP4. On the other hand, the Examples shown below comprise clear instructions allowing the person skilled in the art to recognise which conditions fall within the functional definition (i.e. diseases which are mediated by TLR4, IL1R, COX1/2 and/or RBP4).

For instance, according to the results provided in the present invention, the following inflammatory diseases, or other diseases, such as infectious diseases causing an inflammatory response, could be prevented and/or treated:
- Diseases or conditions which may be treated through the blockade of TLR4 with the compounds of the present invention: Sepsis, SARS-CoV infection, Human immunodeficiency virus (HIV) infection, Influenza A virus (IAV) infection, Respiratory syncytial virus (RSV) infection, Dengue (DENV) infection, Ebola virus (EBOV) infection, Chlamydia infection, Osteoarthritis (OA), Rheumatoid arthritis (RA), Ankylosing spondylitis, Gout, Heterotopic ossification, Fibrodysplasia ossificans progressive, Cardiovascular diseases, Neuroinflammation, Traumatic brain injury (TBI), Systemic lupus erythematosus (SLE), Psoriasis/Psoriatic arthritis and/or IBD (inflammatory bowel disease).
- Diseases or conditions which may be treated through the blockade of IL1R with the compounds of the present invention:
   ∘ Autoinflammatory periodic fever syndromes from two years of age:
      ▪ Cryopyrin-associated periodic syndromes (CAPS):
         - Muckle-Wells syndrome.
         - Neonatal Multisystem Inflammatory Disease (NOMID).
         - Chronic Infantile Cutaneous and Joint Neurological Syndrome (CINCA).
         - Familial Cold Autoinflammatory Syndrome (FCAS).
         - Familial Cold Urticaria (FCU).
      ▪ Tumor necrosis factor receptor-associated periodic syndrome (TRAPS).
      ▪ Hyperimmunoglobulin D syndrome (HIDS).
      ▪ Mevalonate kinase deficiency (MKD).
      ▪ Familial Mediterranean Fever (FMF).
   ∘ Adult Still's disease and systemic juvenile idiopathic arthritis
   ∘ Gouty arthritis
   ∘ Rheumatoid Arthritis (RA)
   ∘ COVID-19
   ∘ Periodic febrile syndromes from 8 months of age:
      ▪ Cryopyrin-associated syndromes (CAPS):
         - Muckle-Wells syndrome
         - Neonatal Multisystem Inflammatory Disease (NOMID)
         - Chronic Infantile Cutaneous and Joint Neurological Syndrome (CINCA)
         - Familial Cold Autoinflammatory Syndrome (FCAS)
         - Familial Cold Urticaria (FCU)
      ▪ Familial Mediterranean Fever (FMF)
   ∘ Adult Still's disease or systemic juvenile idiopathic arthritis
   ∘ Normal immune responses regulation
   ∘ Sepsis
   ∘ Human immunodeficiency virus (HIV)
   ∘ Osteoarthritis (OA)
   ∘ Ankylosing spondylitis
   ∘ Pseudogout
   ∘ Heterotopic ossification (HO)
   ∘ Cardiovascular diseases (atherosclerosis, among others)-IL-1 affects all components of the vessel wall and cardiomyocytes and, accordingly, it plays an important role in atherosclerosis and its complications including myocardial infarction.
   • Diseases or conditions which may be treated through the blockade of COX1/2 with the compounds of the present invention: Atopic dermatitis, Ankylosing spondylitis, Osteoarthritis (OA), Rheumatoid arthritis (RA), Gout, Heterotopic ossification (HO), Fibrodysplasia ossificans progressiva (FOP), Pain/Edema, Neoplasia and/or Fever.
   • Diseases or conditions which may be treated through reduction of the expression of RBP4 with the compounds of the present invention: Ovarian Cancer, Insulin resistance, Inflammation, Retinopathies, Fatty liver and/or Hypertension.

In a preferred embodiment, the present invention refers to the compounds of the invention, or salts derived thereof, for use in the prevention and/or treatment of inflammatory diseases mediated by TLR4, IL1R, COX1/2 and/or RBP4, for example OA.

In a preferred embodiment, the compounds of the invention, or salts derived thereof, is administered topically, orally or by means of an intraarticular, subcutaneous, intra-venous or muscular injection.

The second embodiment of the present invention refers to a pharmaceutical composition (hereinafter *pharmaceutical composition of the invention*) comprising the compounds of the invention, or salts derived thereof, for use in the prevention and/or treatment of diseases mediated by TLR4, IL1R, COX1/2 and/or RBP4.

In a preferred embodiment, the present invention refers to the pharmaceutical composition of the invention for use in the prevention and/or treatment of inflammatory diseases mediated by TLR4, IL1R, COX1/2 and/or RBP4, for example OA.

In a preferred embodiment, the pharmaceutical composition of the invention is administered topically, orally or by means of an intraarticular, subcutaneous, intra-venous or muscular injection.

The third embodiment of the present invention refers to a method for the prevention and/or treatment of diseases mediated by TLR4, IL1R, COX1/2 and/or RBP4, preferably inflammatory diseases mediated by TLR4, IL1R, COX1/2 and/or RBP4, most preferably OA, which comprises administering to the patient a therapeutically effective dose or amount of the compound or pharmaceutical composition of the invention.

The compounds of the invention may be used alone or in combination with other therapies, either to complement their action or to reduce the concentration (and associated side effects) of a given drug.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of' is meant including, and limited to, whatever follows the phrase "consisting of'. Thus, the phrase "consisting of' indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Brief description of the figures

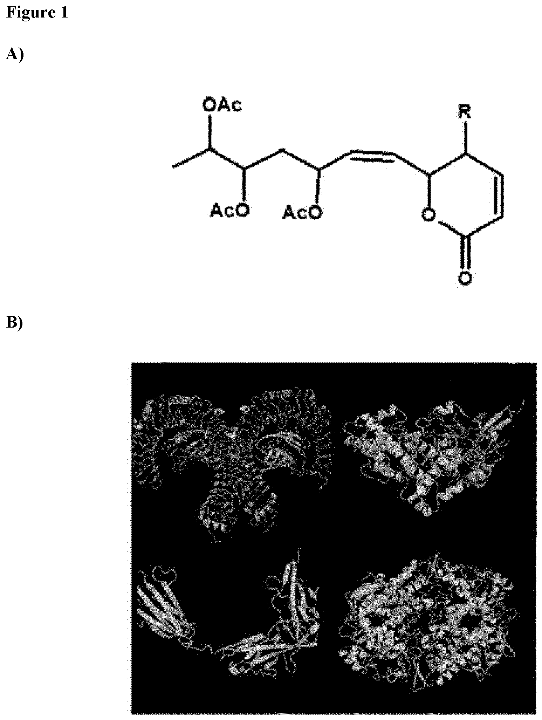

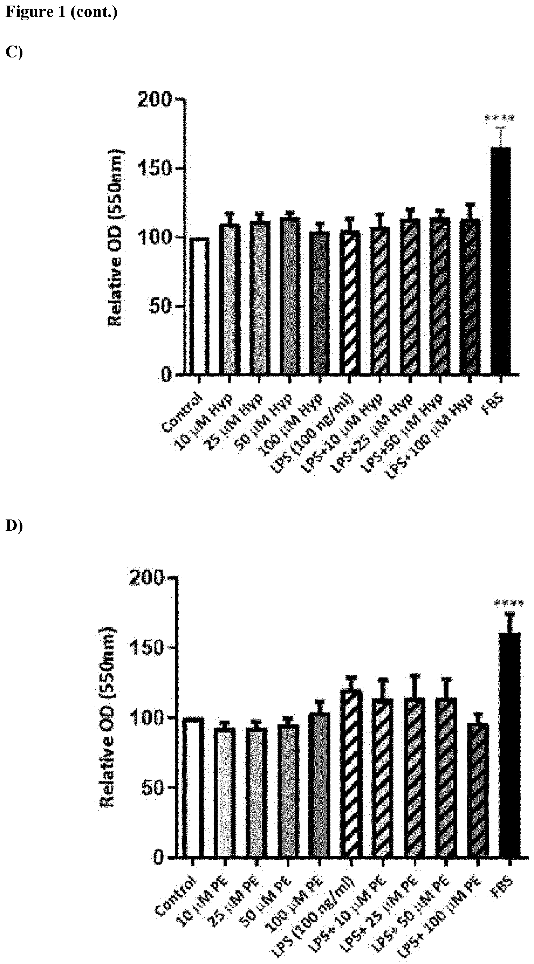

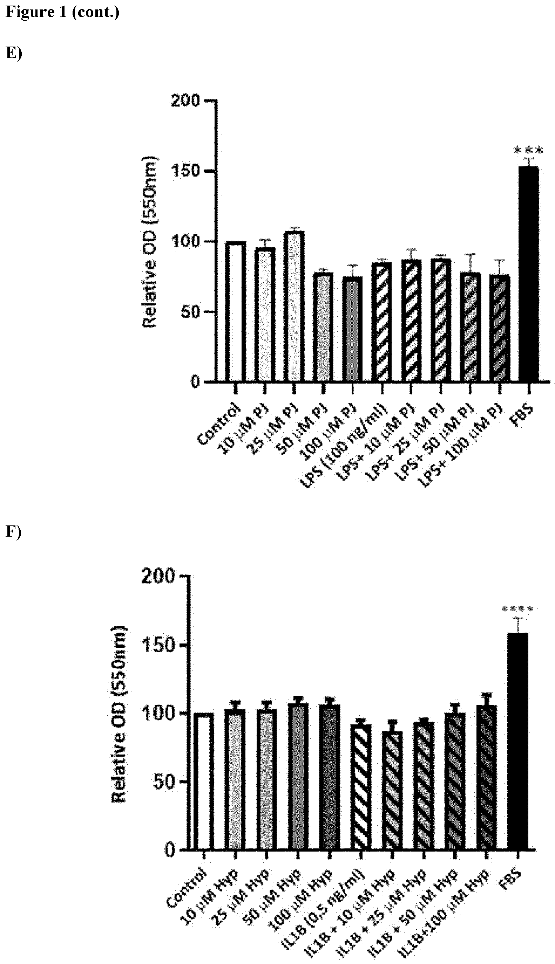

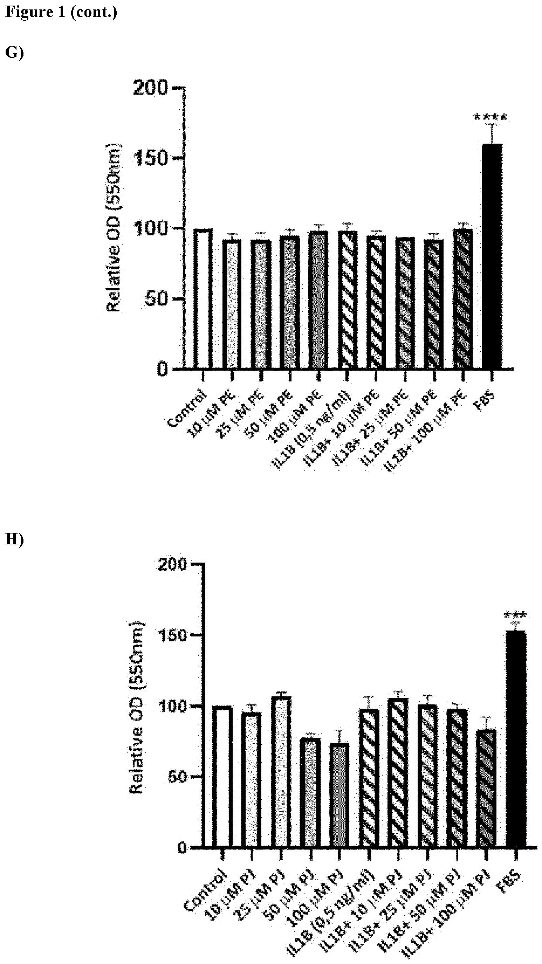

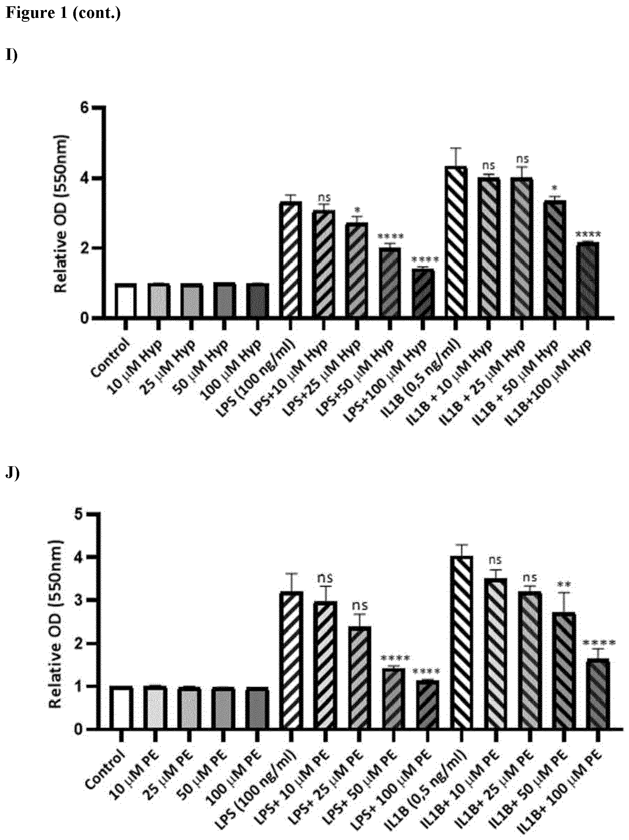

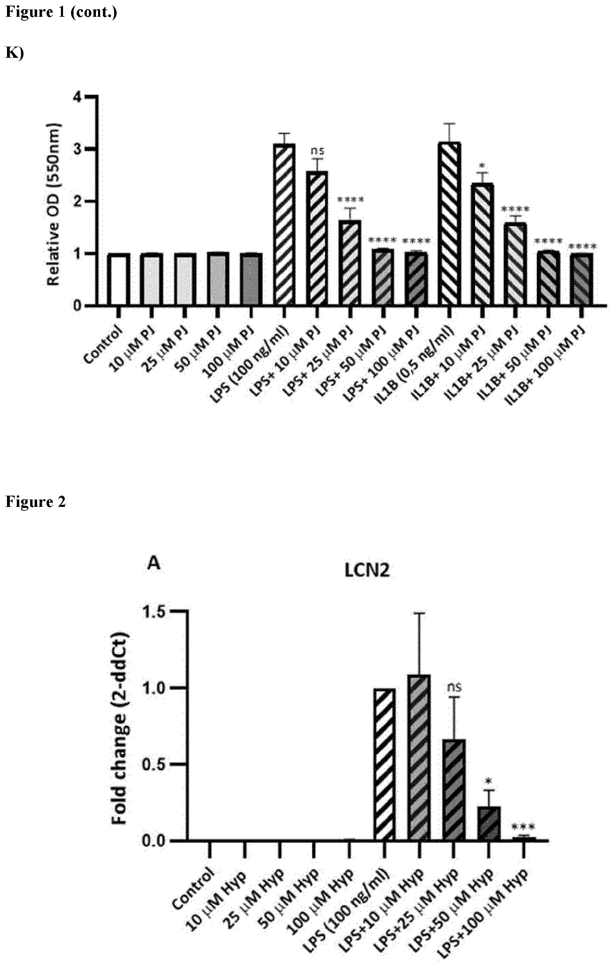

**Figure 1. (A)** Chemical structures of the compounds of the invention: Pectinolide J (R=OH). Hyptolide (R=H). Pectinolide E (R=OAc). **(B)** *In silico* conformational diversity analysis of crystallographic structures. Binding affinity of the compounds of the invention against the key inflammatory targets (TLR4, IL1R, COX1 and COX2). **(C-H)** Effect of the compounds of the invention on cell viability ATDC5 cells (MTT assay). Values are the mean and SEM of at least 3 independent experiments. The Fetal bovine serum (FBS) acts as a positive control. Statistical significance has been determined by One-Way ANOVA. **(I-K)** The compounds of the invention suppress lipopolysaccharide (LPS)-induced or (IL1B)-induced nitric oxide (NO) production. ATDC5 cells (1×10⁵) were stimulated with LPS (100 ng/ml) or IL1β mouse (0,5 ng/ml) and co-stimulated with several doses of Hyp **(I)**, PE **(J)** and PJ **(K)** for 24 hours. NO concentration was determined using Griess reaction. Values are the mean and SEM of at least 3 independent experiments. Statistical significance has been determined by One-Way ANOVA.

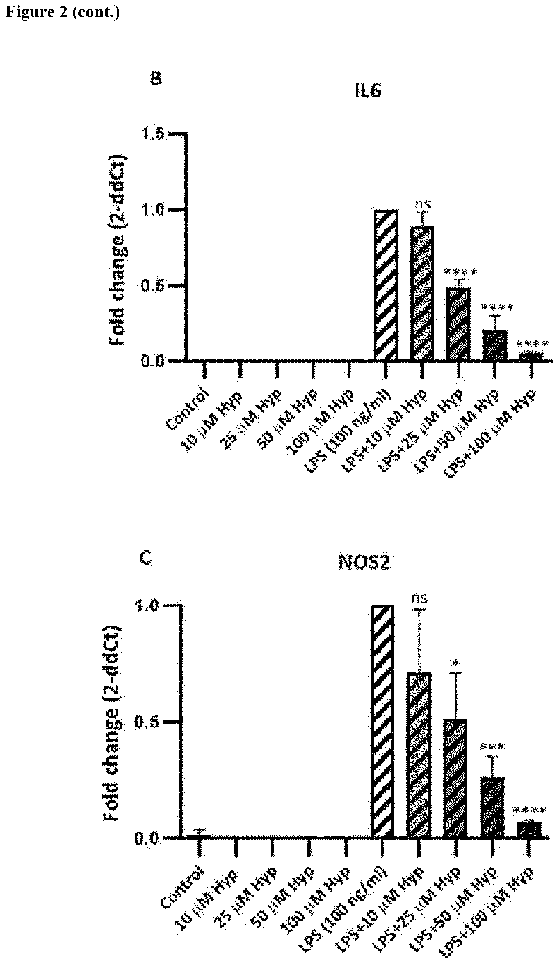

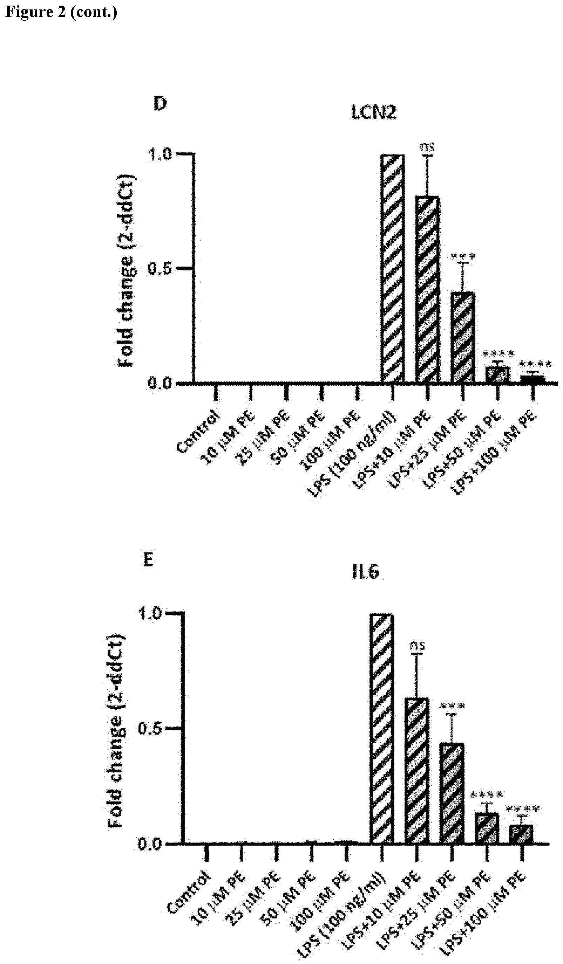

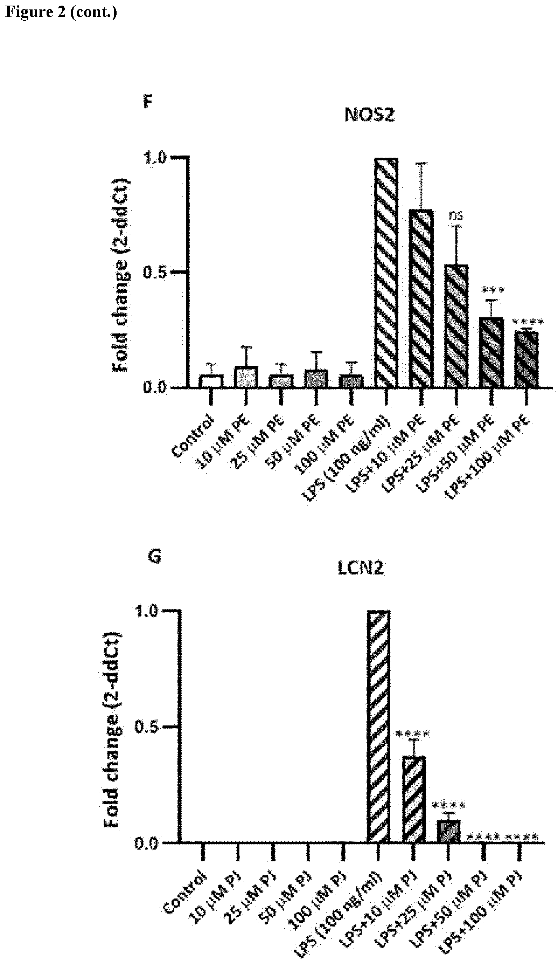

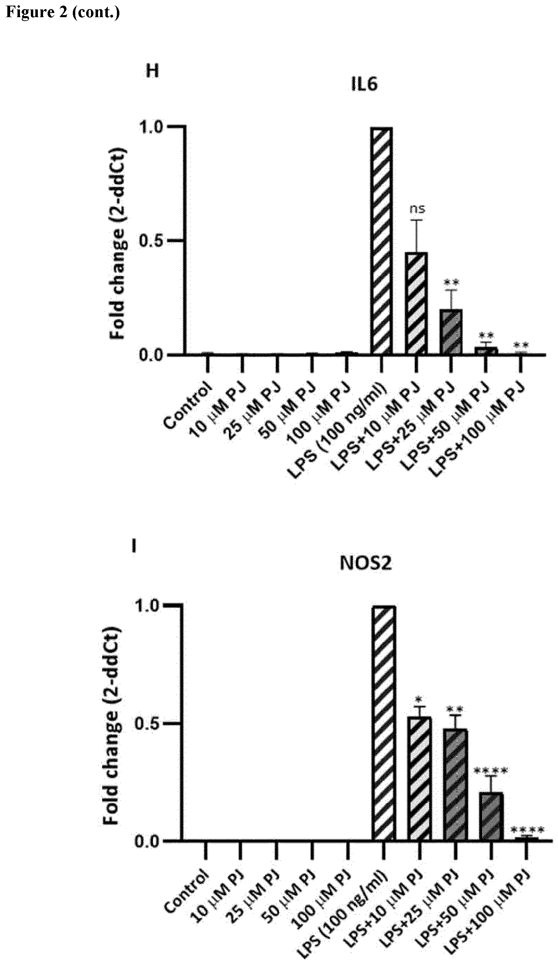

**Figure 2****. Effect of the compounds of the invention on pro-inflammatory TLR4-induced mRNA expression.** ATDC5 cells (1×10⁵) were stimulated with LPS (100 ng/ml) and co-stimulated with several doses of the compounds of the invention. The mRNA expression of lipocalin-2 (LCN2), interleukin-6 (IL-6) and nitric oxide synthase 2 (NOS2) were determined by performing real-time PCR (RT-PCR). Values are the mean and SEM of at least 3 independent experiments. Statistical significance has been determined by One-Way ANOVA.

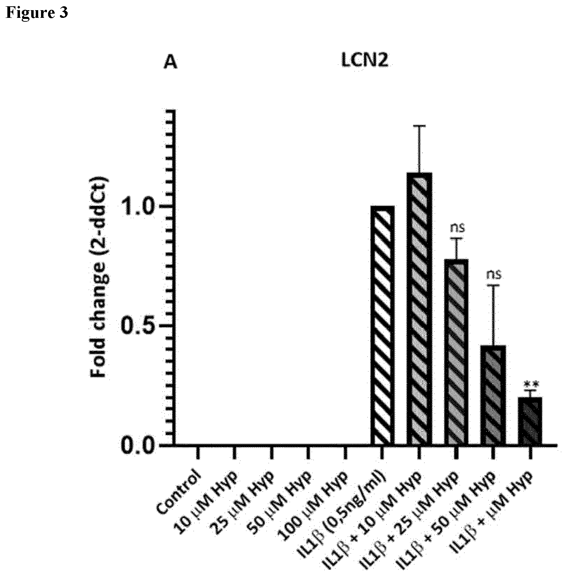

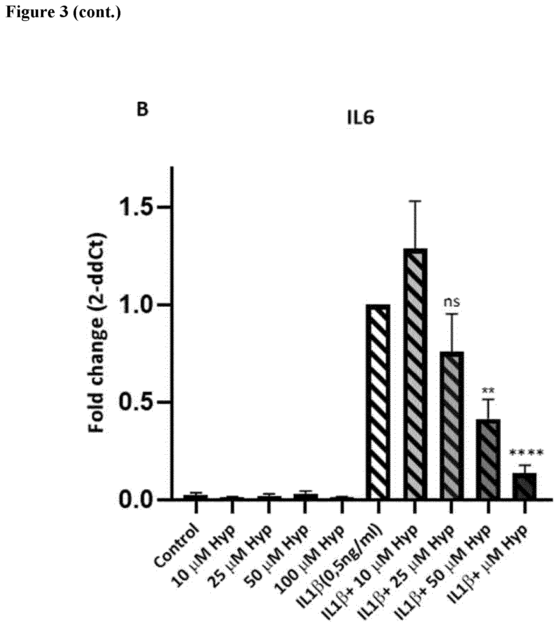

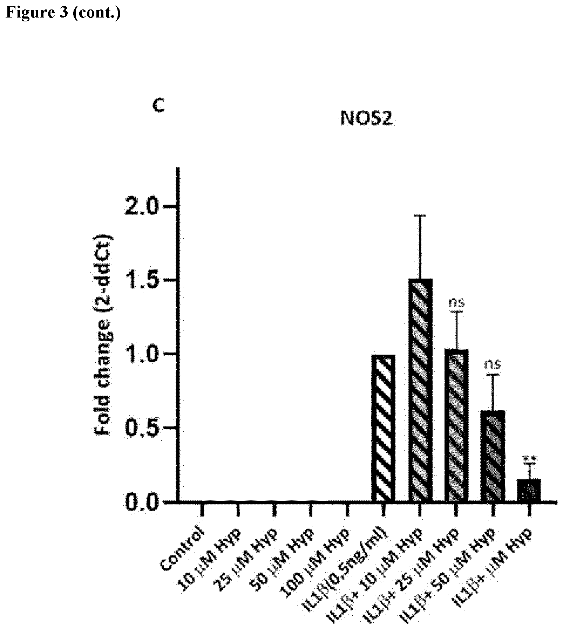

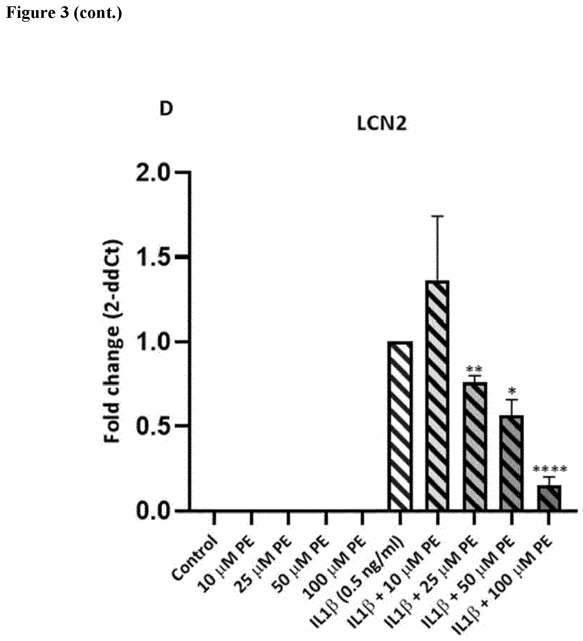

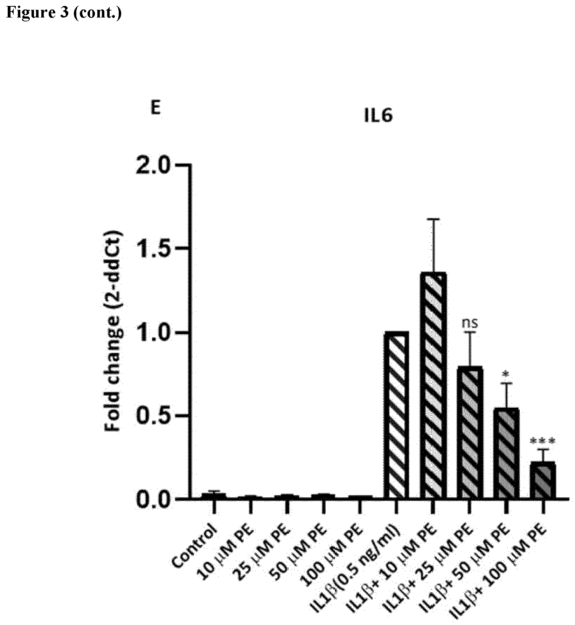

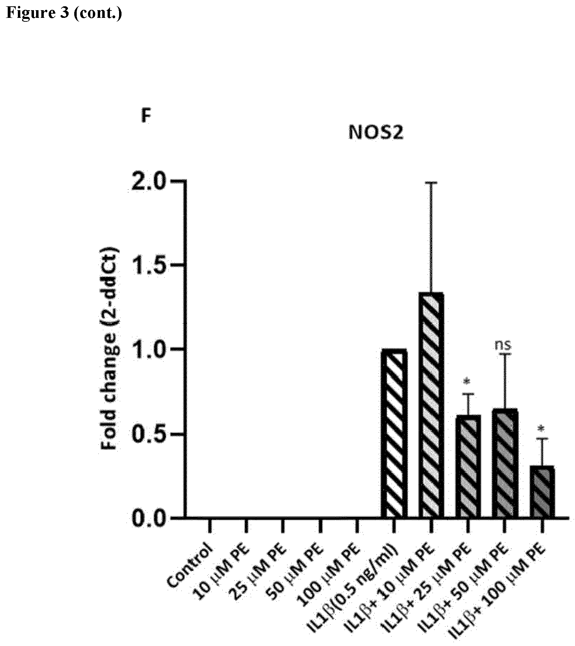

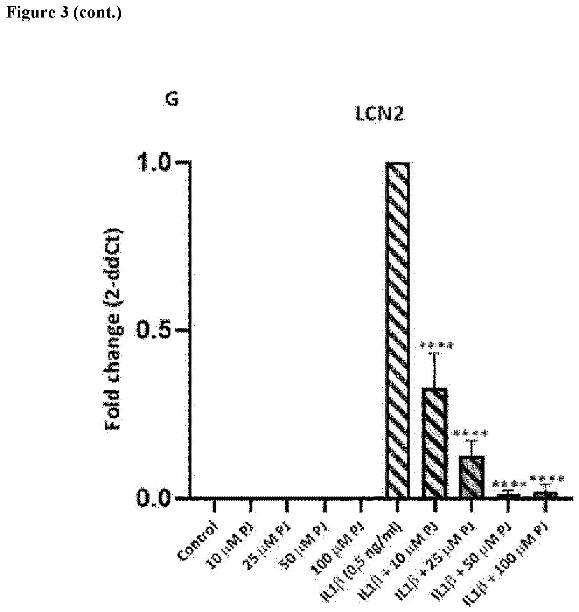

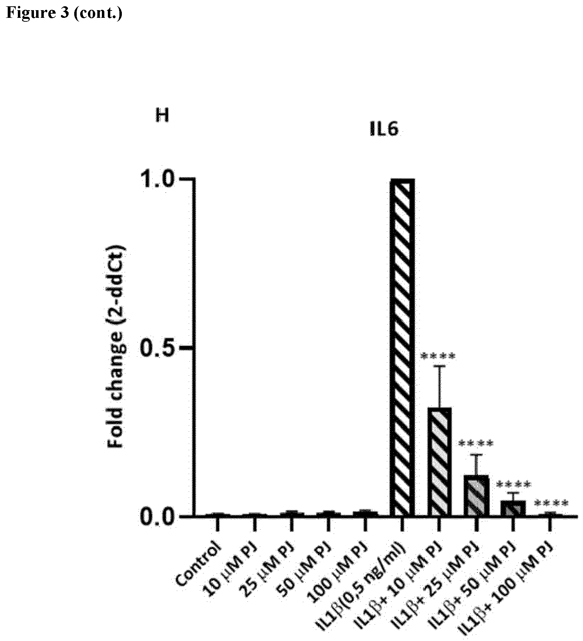

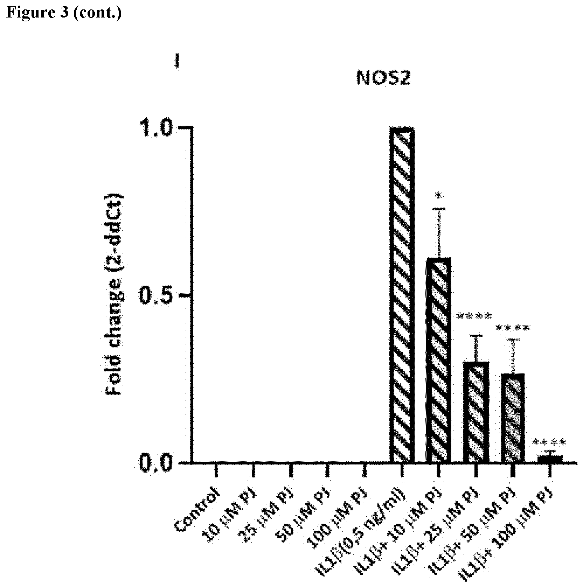

**Figure 3****. Effect of the compounds of the invention on pro-inflammatory IL1-induced mRNA expression.** ATDC5 cells (1×10⁵) were stimulated with IL1β (0,5 ng/ml) and co-stimulated with several doses of the compounds of the invention. The mRNA expression of lipocalin-2 (LCN2), interleukin-6 (IL-6) and nitric oxide synthase 2 (NOS2) were determined by performing real-time PCR (RT-PCR). Values are the mean and SEM of at least 3 independent experiments. Statistical significance has been determined by One-Way ANOVA.

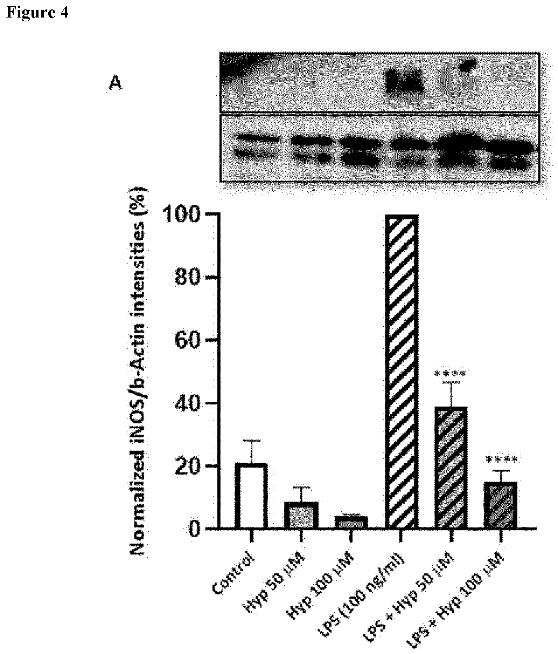

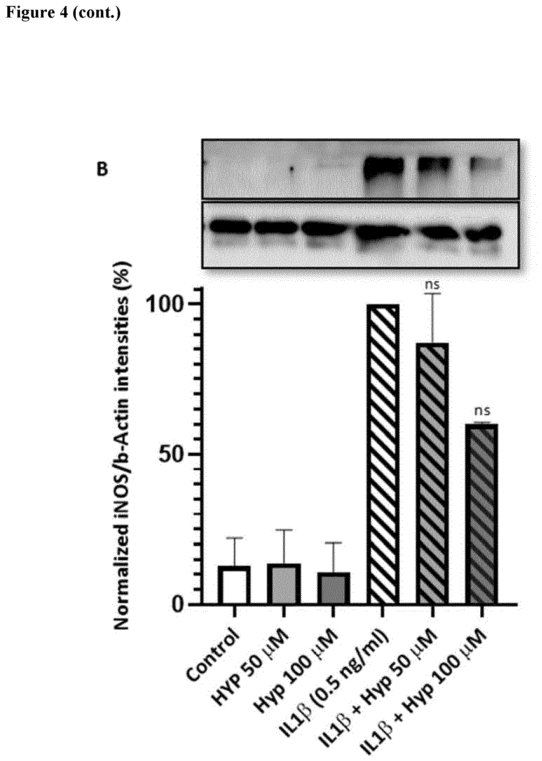

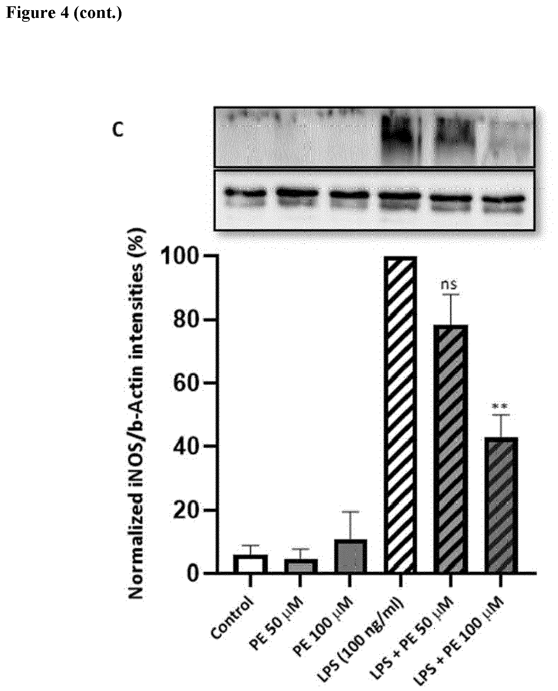

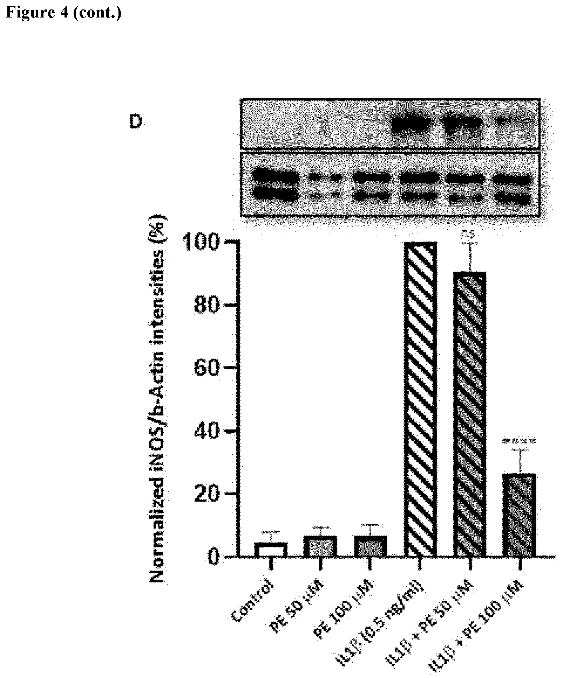

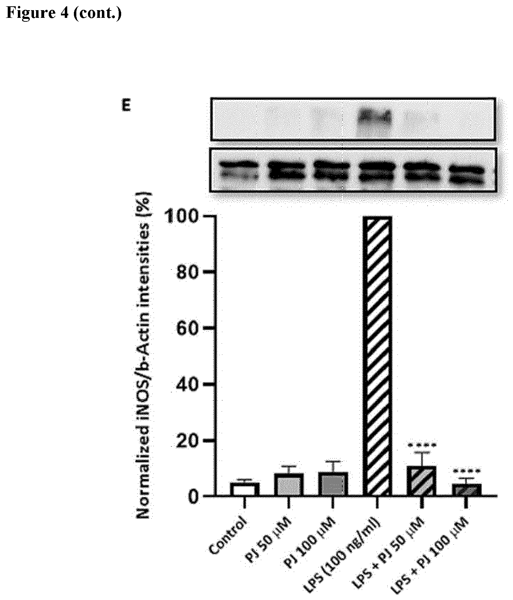

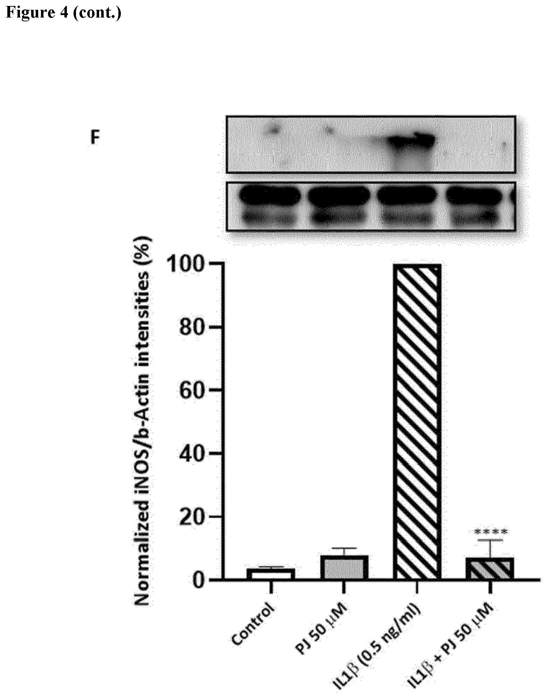

**Figure 4****. Effect of the compounds of the invention on pro-inflammatory TLR4- IL1-induced protein expression. (A-F)** Blots were performed with protein lysates from ATDC5 cells treated for 24 h with the compounds of the invention and LPS (100 ng/ml) or IL1β (0,5 ng/ml) to detect NOS2 and b-actin (an internal standard). Values are the mean and SEM of at least 3 independent experiments. Statistical significance has been determined by One-Way ANOVA.

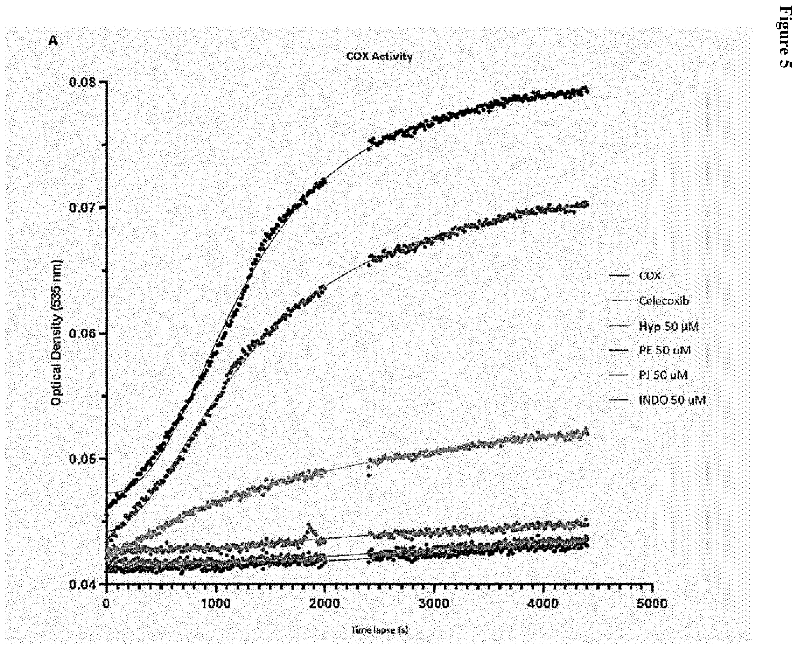

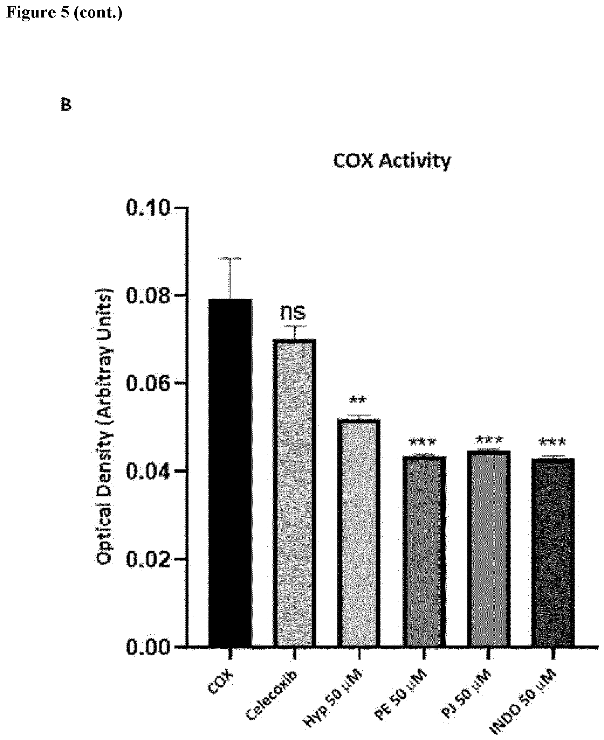

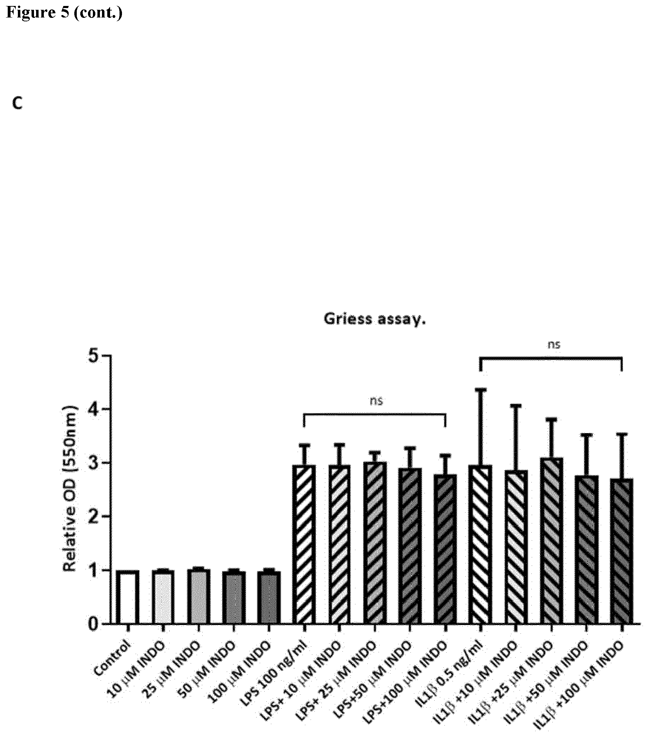

**Figure 5****. Effects of the compounds of the invention on cyclooxygenase activity (A)(B). Indomethacin did not suppress lipopolysaccharide (LPS)-induced or (IL1β)-induced nitric oxide (NO) production (C).** Statistical significance has been determined by One-Way ANOVA.

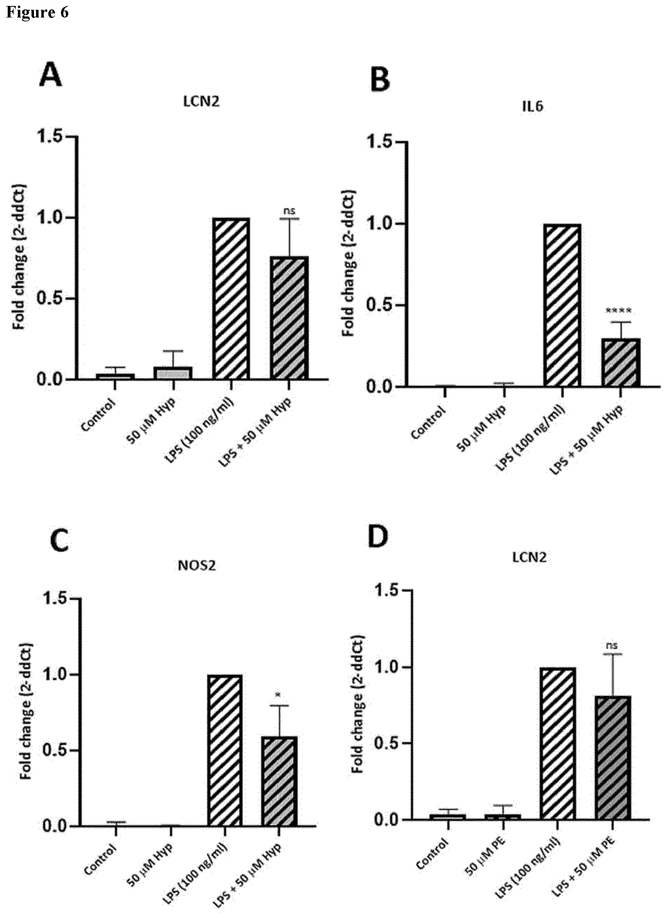

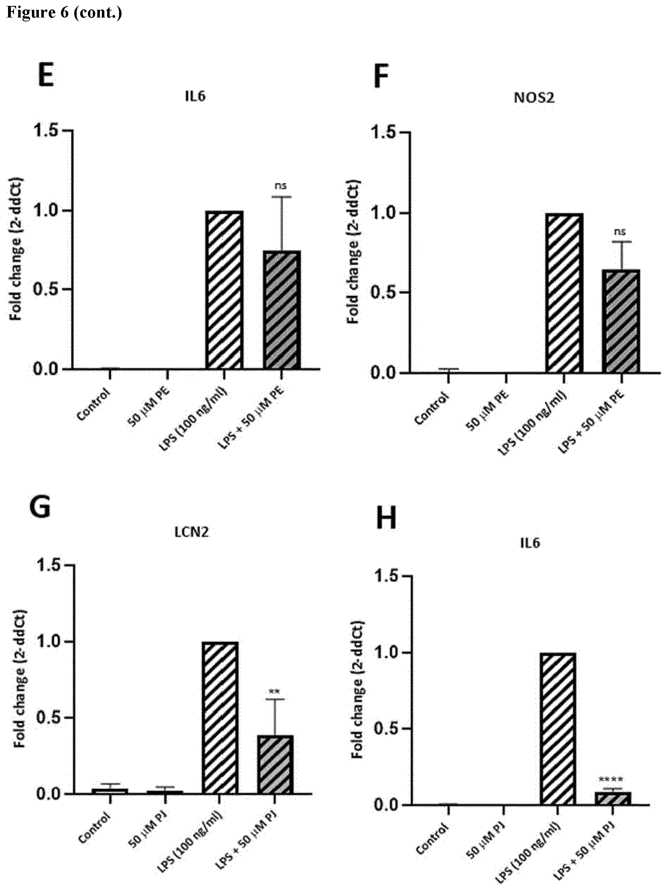

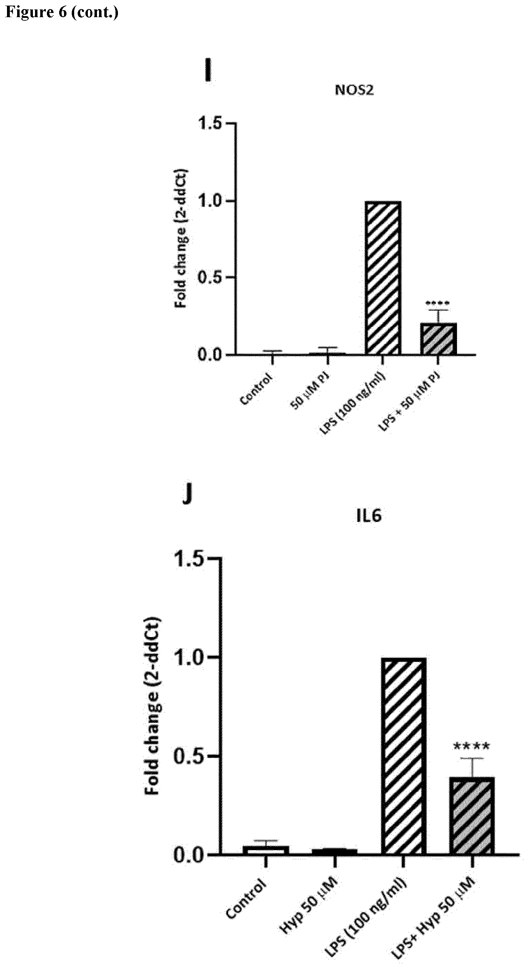

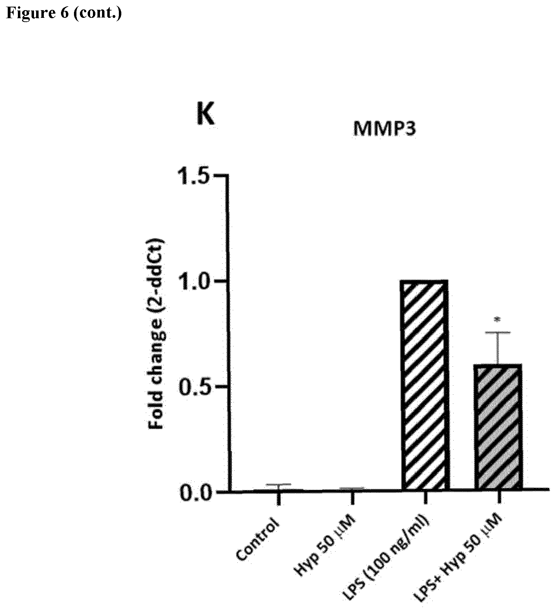

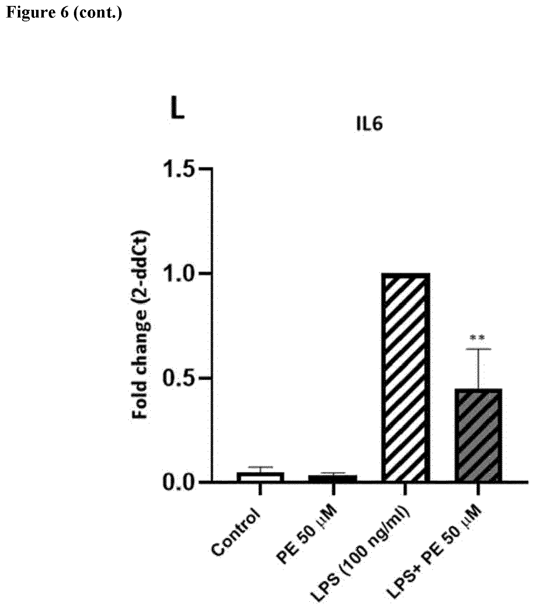

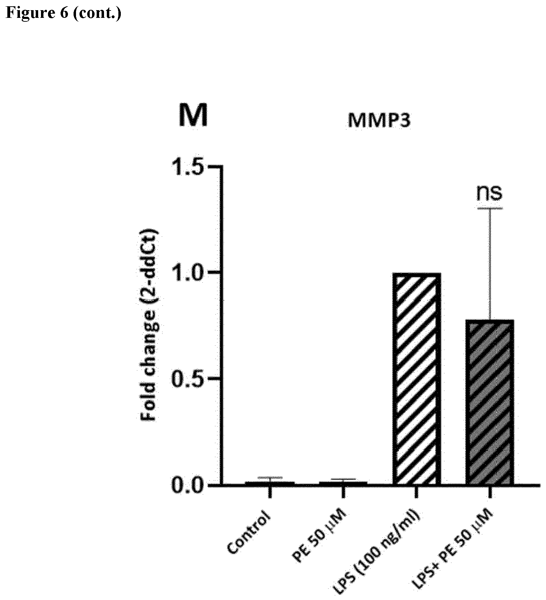

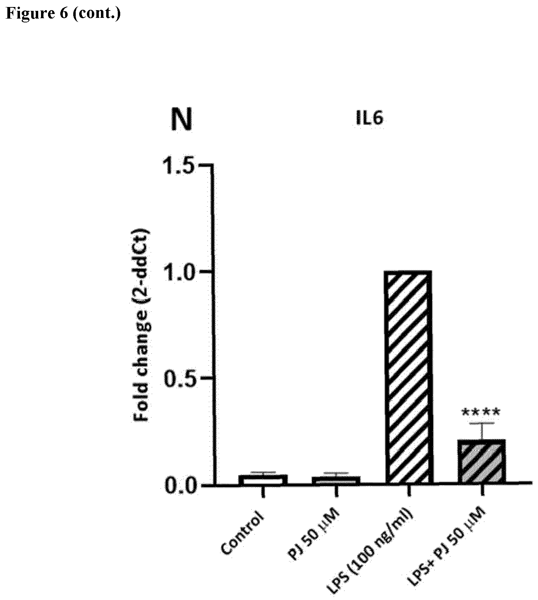

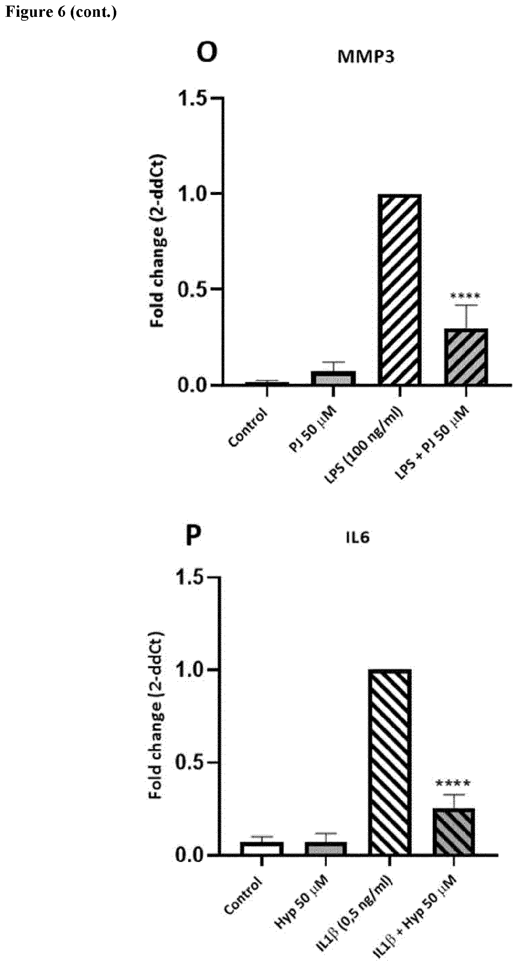

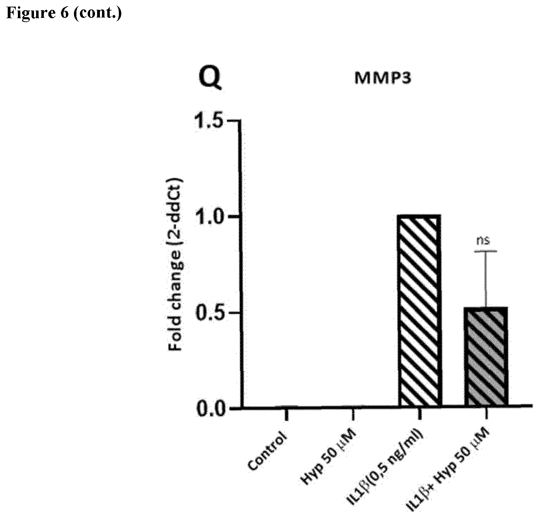

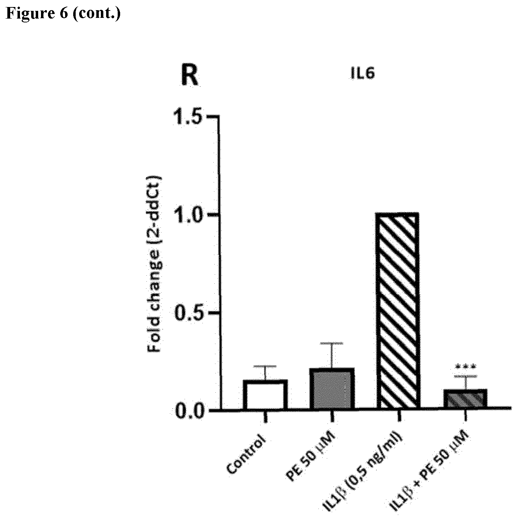

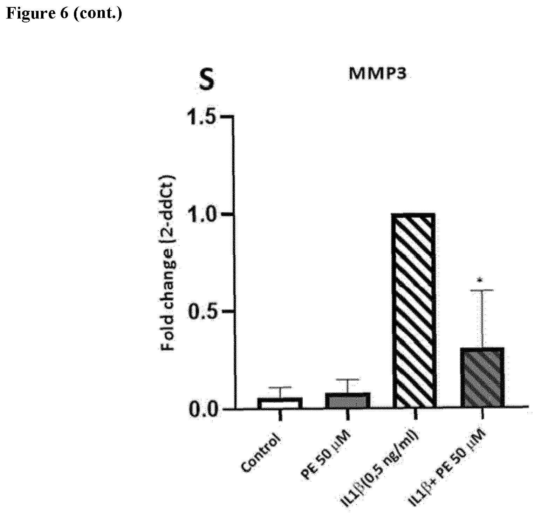

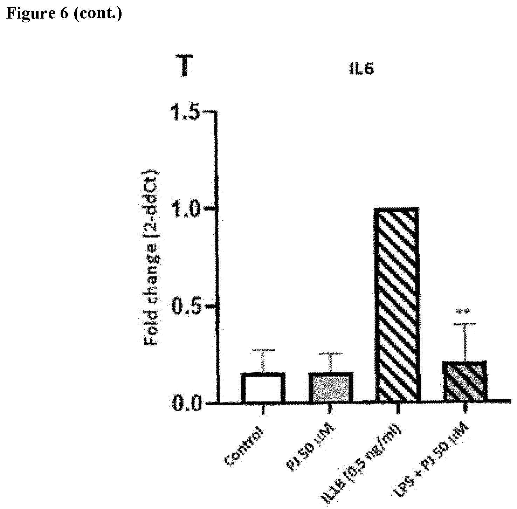

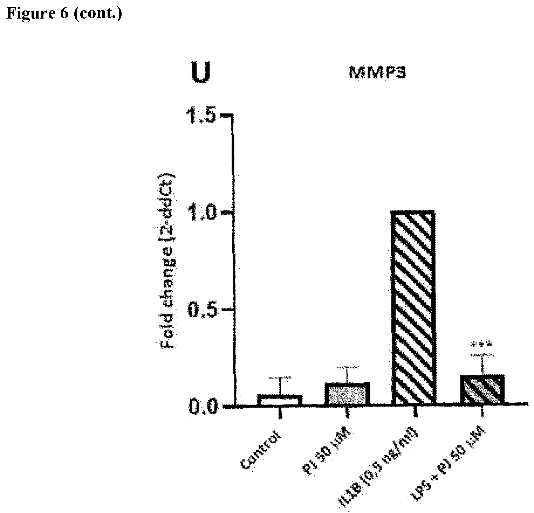

**Figure 6****. The compounds of the invention blocked TLR4- IL1R-mediated innate immune responses in primary human osteoarthritis chondrocytes and osteoblasts.** (A-I) Primary chondrocytes were stimulated with LPS (100 ng/ml) and co-stimulated with 50 µM of the compounds of the invention. Values are the mean and SEM of at least 3 independent experiments. Statistical significance has been determined by One-Way ANOVA. **(J-O)** Primary osteoblasts were stimulated with LPS (100 ng/ ml) and co-stimulated with 50 µM of the compounds of the invention. The pro-inflammatory and catabolic mRNA expression was determined by performing real-time PCR (RT-PCR). Values are the mean and SEM of at least 3 independent experiments. Statistical significance has been determined by One-Way ANOVA. **(P-U)** Primary osteoblasts were stimulated with IL1β (0,5 ng/ ml) and co-stimulated with 50 µM of the compounds of the invention. The pro-inflammatory and catabolic mRNA expression was determined by performing real-time PCR (RT-PCR). Values are the mean and SEM of at least 3 independent experiments. Statistical significance has been determined by One-Way ANOVA.

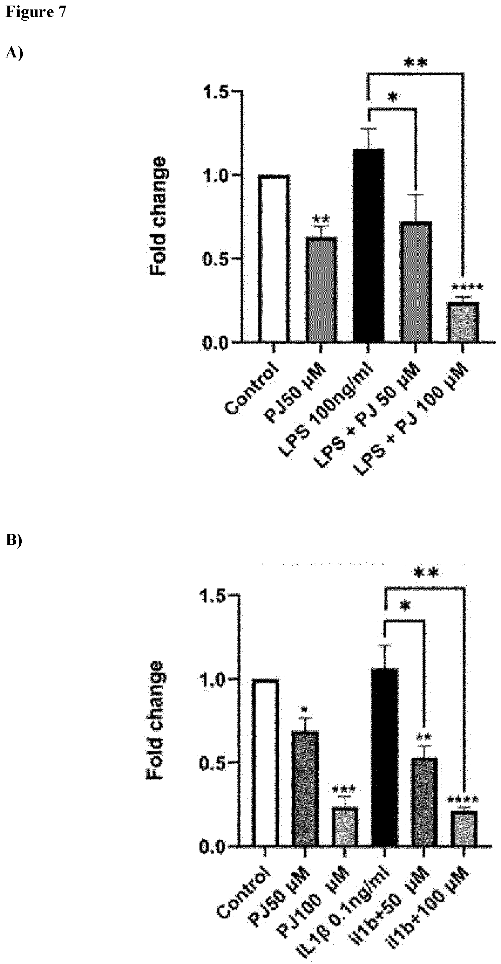

**Figure 7****.** Inhibition of RBP4 expression by PJ (50 and 100 µM of PJ) in the presence or absence of inflammatory stimuli (LPS and IL1β). Values are the mean and SEM of at least 3 independent experiments. Statistical significance has been determined by One-Way ANOVA.

### Detailed description of the invention

The present invention is illustrated by means of the Examples set below without the intention of limiting its scope of protection.

### Example 1. Materials and methods

### Example 1.1 General experimental procedures

Column chromatography was performed on silica gel (70-230 mesh, Macherey-Nagel) and fractions were monitored by TLC on silica gel plates (GF254, Merck), using the vanillin-sulfuric acid as a visualization reagent. HPLC (Shimadzu, Prominence model) was performed using LC-6AD pumps coupled with analytical or semi-preparative Waters C18 columns (250 × 4.6 and 250 × 21.2 mm, respectively). The HPLC system employed an SPD-20A UV-Vis detector. 1H and 13C NMR spectra were obtained using a Bruker AVANCE-III 11.74 Tesla (499.87 MHz for 1H and 125 MHz for 13C) in CD3OH solvent and SiMe4 as an internal standard. MS data were recorded on a Waters Xevo QTof MS. Absorbance data and optic rotations were determined in Cary 50 UV-Vis spectrophotometer and Perkin Elmer model 341 Polarimeter, respectively. All solvents used are of commercial HPLC grade.

### Example 1.2. Plant material

*Hyptis pectinata (L.) Poit* was collected in September 2015. For the extraction process, the inflorescences were dried in an oven with air renewal and circulation at 40°C until complete dehydration (4 days). Then, the vegetal material was triturated to yield a fine powder.

### Example 1.3. Extraction and isolation of the compounds of the invention

The powdered inflorescence (0.88 kg) was extracted with ethanol (3 × 4.4 L) at room temperature (25°C) during 10 minutes for each cycle in high-performance disperser (TE-102 Turratec^{®}, Tecnal, Brazil) to 21,000 rpm. After filtration, the solvent was evaporated under reduced pressure in a rotary vacuum evaporator at 40°C. The resulting extract (62.53 g) was dissolved in EtOH-H2O mixture (6:4, v/v) and extracted with CH₂Cl₂ (3 × 200 mL). The CH₂Cl₂ layer was passed through the Na2SO4 and evaporated under reduced pressure at 40°C, which afforded a dark-coloured gum (HPF1D, 57.84 g). Then, 20.00 g of gum was chromatographed on a silica gel (70-230 mesh) column (47 cm × 6 cm i.d.) with a gradient system of hexane-CH₂Cl₂-Me2CO (6:2:2, 500 mL; 4:4:2, 650 mL; 4:2:4, 2650 mL) to yield seventy-six sub-fractions (50 mL each). Some fractions were combined after monitoring by TLC resulting in eight sub-fractions (HPF1D-1, HPF1D-2, HPF1D-3, HPF1D-4, HPF1D-5, HPF1D-6, HPF1D-7, HPF1D-8). HPF1D-4 (1.2 g) was performed by HPLC analytical scale using reverse phase column (250 × 4.6 mm, 10 µ) at 25°C, mobile phase MeOH-H2O (50:50), detection wavelength at 210 nm and the flow rate at 1.0 mL/min. Then, the compounds 1 (65.7 mg), 2 (23.9 mg) and 3 (123.9 mg) were isolated by semipreparative HPLC on a Waters column (250 × 21.2 mm, 10) at 25°C, and detected at 210 nm at a flow rate of 16.0 mL/min using a mobile phase MeOH-H₂O (50:50). The retention time for 1, 2 and 3 were 13.3, 20.6 and 27.0 min, respectively.

### Example 1.4. Cell culture

ATDC5 murine chondrogenic cell line was obtained from RIKEN Cell Bank and cultured in DMEM/Ham's F-12 medium supplemented with 5% FBS, 2% glutamine, and 50 U/mL of penicillin and 50 µg/ml of streptomycin, 10 µg/mL human transferrin and 3 × 10-8 M sodium selenite.

### Example 1.5. Reagents

Foetal bovine serum (FBS), DMEM F-12 medium, antibiotics, trypsin, 1-glutamine, sodium selenite, methyl- thiazolyl-tetrazolium (MTT), transferrin, LPS (E. coli O127:B8) and primers (HPRT, LCN2, IL6, NOS2, and MMP3) were purchased from Sigma-Aldrich (St. Louis, MO, USA). IL-1β was purchased from Peprotech (NJ, USA).

DNAase I, buffer 10x was purchased from Lucigen (A Coruña, Spain). Retro transcription kit (RT) and ultrapure water were purchased from Applied Biosystems Thermo Fisher (Waltham, MA, USA). To do PCRs, master mix was purchased from BioRad (Alcobendas, Madrid, Spain).

Primary antibodies (iNOS and b-actin) were purchased from Applied Biosystems Thermo Fisher (Waltham, MA, USA). Secondary antibodies (IG mouse and IG rabbit) were purchased from Jackson Laboratories (Jackson ImmunoResearch Laboratories Inc., USA).

### Example 1.6. Molecular docking simulations

The compound structural data were obtained from a Protein Data Bank. Molecular docking simulations were conducted using Auto Dock Vina Software and the ensemble docking method. This software led to study the binding affinity between ligand (receptor) and protein (the compounds of the invention). This software showed the predicted and experimental free energies of binding for the data set (kcal/mol). A threshold criterion (docking score>-5.0 kcal/mol) was set to select the strongest interactions.

### Example 1.7. Cell treatments

ATDC-5 cells were seeded in 12-well plates. Cells were seeded in a monolayer. After adhesion and incubation overnight in serum-free conditions (starvation), ATDC5 cells were stimulated for 24 h with LPS [100 ng/ml] or IL-1β [0,5 ng/ml] in the presence or absence of the compounds of the invention [10-100 µM].

### Example 1.8. Cell viability

Viability was tested using the MTT reagent as previously described. Briefly, 8 × 10³ cells/well were plated in 96-well plates and treated as described above. Then, the cells were incubated for 4 h with MTT reagent. After formazan salt was dissolved, absorbance was measured at 550 nm in a microplate reader.

### Example 1.9. Nitrite assay

As described above, 1×10⁵ ATDC5 cells were seeded and treated for 24 h. Nitrite accumulation was determined in the culture medium using the Griess reaction. As previously described, after diazonium salt was dissolved, absorbance was measured at 550 nm in a microplate reader.

### Example 1.10. Cyclooxygenase (COX) Activity Assay

The anti-inflammatory activity of the compounds of the invention was tested in terms of COX inhibition. The COX inhibition effect of the compounds of the invention was estimated using a Fluorometric Cyclooxygenase COX activity assay (Abcam, ab204699) according to the manufacturer's protocol.

After the addition of arachidonic acid (COX substrate), the enzymatic reaction led to the production of a fluorescent molecule (resorufin dye λ=535 nm), that could be monitored in kinetic mode (4 reads per min) for 60 min at room temperature (Epoch 2 de Bio Tek).

### Example 1.11. RNA extraction and RT-PCR

RNA was extracted using E.Z.N.A solation reagent (Omega) according to manufacturer's protocol. Relative quantitation was performed using the ΔΔC Comparative Method and the MxPro software (Stratagene, CA, USA) as the ratio of each gene to the expression of the housekeeping gene. Data are shown as mean ± S.E.M (error bars) of at least three independent experiments and represented as fold-change vs. controls.

### Example 1.12. Protein Extraction and western blotting

Whole-cell protein extraction was performed using lysis buffer (RIPA Buffer). Immunoblots were incubated with the appropriated antibodies [INOS (rabbit polyclonal antibody) diluted 1:1000 (Thermo Fisher Scientific, MA, USA), IG (anti-rabbit antibody) diluted 1:5000 (Jackson ImmunoResearch Laboratories Inc., USA), b-actin (mouse polyclonal antibody) (Thermo Fisher Scientific, MA, USA) diluted 1:6500 and IG (anti-mouse antibody) diluted 1:10000] (Jackson ImmunoResearch Laboratories Inc., USA).

Then, all immunoblots were visualized using an Immobilon Western kit (Millipore, MA, USA). Blots densitometries were generated using a *ChemiDoc MP Imaging System* and analysed using Image J software (National Institutes of Health, Bethesda, MD, USA).

### Example 1.12. Statistical analysis

Data were reported as mean ± SEM for at least three independent experiments. Statistical analyses were performed by analysis of variance followed by the One-Way Anova multiple comparison test or t-test analysis using Prism statistical analysis (GraphPad Software). p < 0.05 was considered significant.

### Example 2. Results

### Example 2.1. Isolation and characterization of the compounds of the invention

We decided to isolate three structurally related pectinolides, which only differ in one substituent at position 5 (R). This structural similarity was used to explore the structure-activity relationship of these compounds. Specifically, Hyp contains a Hydrogen (R=H), Pectinolide E contains an acetoxy group (R=OAc), and Pectinolide J contains a hydroxyl group (R=OH) (**Fig. 1A**).

### Example 2.2. Molecular docking of the compounds of the invention on key Innate immune targets.

In order to investigate the potential biological activity of the compounds of the invention, we decided to run a docking analysis test against key inflammatory targets (TLR4, IL1R, COX1, and COX2) (**Fig. 1B**)**.** Docking scores obtained, represented as kcal/mol, revealed a strong bind with the studied targets (**Table 1**).

For TLR4 receptor, docking scores of the compounds of the invention were stronger than the one of LPS (a widely known TLR4 agonist) (**Table 1**). In comparison to HYP, PE and PJ dockings were slightly greater towards this receptor. This difference was not observed between PE and PJ (**Table 1**). The docking analysis was also run against IL1R, another innate immune receptor. In this case, docking scores of the compounds of the invention were similar to Methotrexate (a known IL1R inhibitor). As observed for TLR4, PE and PJ exhibited the highest docking scores, which were significantly greater than HYP docking score (**Table 1**).

Considering the key role of COX enzymes activity in OA inflammatory responses, we further investigated the docking ability of the compounds of the invention against them. Data obtained showed that unlike HYP PE and PJ docking scores were stronger than indomethacin (a well-known COX1/2). To note, Hyp also exhibited a notable docking score against these enzymes.

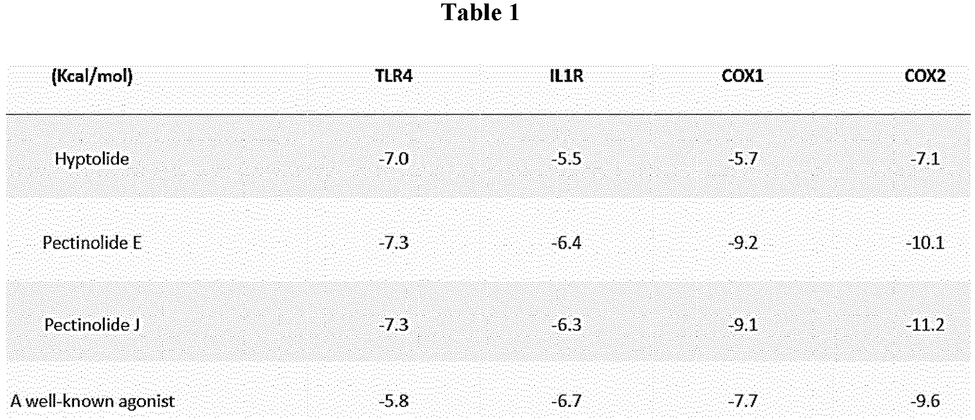

### Example 2.3. Effect of the compounds of the invention on chondrocytes viability.

Aimed to evaluate the potential anti-inflammatory effects of the compounds of the invention without any toxic effect, we evaluated through a 48h MTT assay whether the isolated compounds affected cell viability. As shown in **Figure 1 C-H**, the compounds alone or in combination with inflammatory stimuli (LPS or IL1β) did not significantly affect ATDC5 cell viability.

### Example 2.4. The compounds of the invention suppress NO production in the ATDC5 cell line.

NO is a key inflammatory mediator released upon TLR4 and IL-1R activation. Therefore, to evaluate the potential anti-TLR4 and -IL1R of the compounds of the invention ATDC5 cells were stimulated for 24h with LPS (100 ng/ml) or IL1B (0,5 ng/ml) in the presence or absence of the compounds of the invention. As shown in **Figure 1**, LPS and IL1B increased NO production, determined as nitrite accumulation. In contrast, the compounds of the invention alone did not modulate NO production. However, these compounds were able to significantly reduce NO production induced by LPS and IL1β in a dose-response fashion (**Fig. 1 I-K**). The activity of the compounds of the invention was not even reducing NO production. Specifically, Pectinolide J (R = OH) compound exhibited the maximum inhibitory activity (**Fig 1K**) [10-25 µM]. Nonetheless, all the compounds of the invention significantly reduced the induced NO production above a certain concentration threshold (50 µM).

### Example 2.5. The compounds of the invention suppress the expression of proinflammatory mediators upon TLR4 activation.

Activation of toll-like receptor 4 (TLR4) is associated with innate immune responses (IIRs) in articular tissues (1). Among these responses, TLR4 activation enhances the expression of pro-inflammatory mediators such as lipocalin-2 (LCN2), interleukin-6 (IL6) and nitric oxide synthase 2 (NOS2). To elucidate the anti-TLR4 activity of the compounds of the invention, we examined the LPS-induced mRNA expression of these mediators in the presence of the compounds of the invention. These compounds alone did not modify the expression of LCN2, IL6 and NOS2 after 24h stimulation. However, as shown in **Figure 3**, the expression of LPS-induced IIRs was significantly decreased upon treatment with the compounds of the invention. Above a certain concentration (25 µM) all the compounds were able to significantly decrease the mRNA expression of the inflammatory mediators. Remarkably, PJ compound exhibited the highest anti-inflammatory activity, showing similar results at lower concentrations.

To further validate this result, we evaluated the protein expression of the 250 kDa NOS2 dimer, which is an enzyme highly induced upon TLR4- and IL1R- activation. As shown in **Figure 4**, LPS stimulation induced NOS2 expression in ATDC5 cells. However, this expression was significantly decreased after co-stimulation with the compounds of the invention. Consistent with previous experiments, PJ induced the highest reduction of NOS2 expression (**Fig. 4** **E**).

### Example 2.6. The compounds of the invention suppress the expression of inflammatory mediators upon IL1R activation.

Activation of interleukin-1 receptor (IL1R) is associated with innate immune responses (IIRs) in articular tissues (1). Like TLR4 activation, IL1R activation enhances the expression of LCN2, IL6 NOS2. To elucidate the anti-ILl activity of the compounds of the invention, we examined the IL1β-induced mRNA expression of these mediators in the presence of the compounds of the invention. After 24h stimulation, these compounds alone did not modify the expression of any of the IIRs studied. However, as shown in **Figure 3**, the IL1β-induced expression of LCN2, IL6 and NOS2 was significantly decreased upon treatment with the compounds of the invention. As observed in LPS-treated cells, PJ compound exhibited the maximum inhibitory activity (**Fig 3 A-I**) [10 µM] followed by PE [50 µM] and Hyp [100 µM]. Nonetheless, above a certain concentration threshold (25 µM) all the compounds significantly decrease the mRNA expression of several inflammatory mediators. As performed in the LPS-stimulated ATDC5 cells, to further validate these results we evaluated the protein expression of the 250 kDa NOS2 dimer. As shown in **Figure 4**, IL1β stimulation induced NOS2 expression in ATDC5 cells. However, this expression was significantly decreased after co-stimulation with the compounds of the invention. Similarly, to the effects of PJ on LPS-stimulated cells, it induced the highest reduction of NOS2 expression among the tested compounds (**Fig. 4** **F**).

### Example 2.7. The compounds of the invention reduce the COX 1/2 enzymatic activity.

Cyclooxygenases (COX 1/2) are enzymes highly involved in articular inflammatory processes through their prostaglandin synthase activity. According to the molecular docking results, the compounds of the invention may inhibit these enzymes. Therefore, using a resorufin-based assay, we explored COX 1/2 activity in the presence or absence of the compounds of the invention. As shown in the **Figure 5**, the compounds of the invention significantly reduced COX 1/2-mediated resorufin production. Unlike for Hyp, this effect was comparable to indomethacin activity, a well-known (COX 1/2) inhibitor. To elucidate whether the compounds of the invention show both activities (anti-COX1 and anti-COX2) we also compared the results obtained with the compounds of the invention with the specific COX2 inhibitor (Celecoxib). Consistent with a dual action on COX1/2 enzymes, the inhibitory activity of the compounds of the invention was greater than celecoxib.

According to these results, we decided to investigate if indomethacin also showed anti-TLR4 and anti-IL1R activity on LPS treated chondrocytes. Indomethacin did not significantly inhibit TLR4 nor IL1R-mediated inflammatory responses, suggesting that the blockade of these receptors by the compounds of the invention was independent of their anti-COX1/2 activity.

### Example 2.8. The compounds of the invention blocked TLR4- IL1R- mediated innate immune responses in primary human osteoarthritis chondrocytes and osteoblasts.

To validate these results in a human pathological context, we studied the effects of the compounds of the invention on TLR4- and IL1R-activated primary OA chondrocytes and osteoblasts. According to the results obtained in ATDC5 cells, we selected the concentration 50 µM and co-treated the primary cells with inflammatory stimuli for 24h at the same concentrations [LPS (100 ng/ml), and IL1β (0,5 ng/ml)].

Consistent with the results obtained in ATDC5 cells, after 24h treatment with the compounds of the invention, the inflammatory and catabolic markers genes induced by the inflammatory stimuli (LCN2, NOS2, IL6, MMP3) were significantly reduced in both primary cell cultures (**Fig. 6**). As it was revealed by ATDC5 experiments, PJ showed the maximum anti-inflammatory activity.

### Example 2.9. The compounds of the invention suppress the pro-inflammatory protein RBP4

RBP4 has been described as an adipokine that has been associated with several metabolic disorders such as diabetes, obesity, or metabolic syndrome, it has also been given a relevant role in other diseases characterised by a chronic inflammatory state like arthritis. Accordingly, previous reports have suggested that RBP4 might bind to Toll-like receptor 4 (TLR4). As shown in **Figure 7****,** Pectinolide J (R = OH) downregulates in chondrocytes RBP4 mRNA expression in both basal or inflammatory conditions generated by lipopolysaccharide (LPS) or interleukin 1 beta (IL1β). This is a relevant result because Pectinolide J, apart from blocking TLR4, could also reduce the positive inflammatory effect of RBP4 working as a TLR4 activating agent.

## Claims

1. Compound of Formula (I), or salts derived thereof, wherein **OAc** is an acetyloxy group and **R** may be substituted by **OH**, **H** or **OAc**, 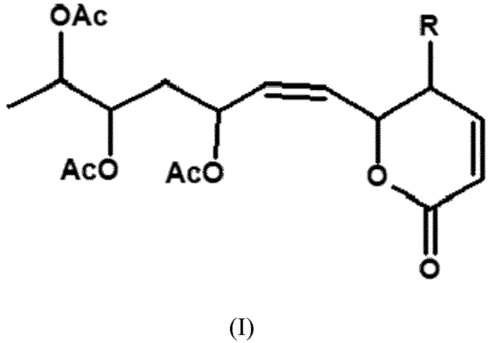 for use in the prevention and/or treatment of diseases mediated by TLR4, IL1R, COX1/2 and/or RBP4.

2. Compound of Formula (I) for use, according to claim 1, in the prevention and/or treatment of inflammatory diseases mediated by TLR4, IL1R, COX1/2 and/or RBP4.

3. Compound of Formula (I) for use, according to any of the claims 1 or 2, in the prevention and/or treatment of osteoarthritis.

4. Compound for use, according to any of the claims 1 to 3, wherein the compound is administered topically, orally or by means of an intraarticular, subcutaneous, intravenous or muscular injection.

5. Pharmaceutical composition comprising a compound of Formula (I), or salts derived thereof, wherein **OAc** is an acetyloxy group and **R** may be substituted by **OH**, **H** or **OAc**, and, optionally, excipients or carriers pharmaceutically acceptable, for use in the prevention and/or treatment of diseases mediated by TLR4, IL1R, COX1/2 and/or RBP4.

6. Pharmaceutical composition for use, according to claim 5, in the prevention and/or treatment of inflammatory diseases.

7. Pharmaceutical composition for use, according to any of the claims 5 or 6, in the prevention and/or treatment of osteoarthritis.

8. Pharmaceutical composition for use, according to any of the claims 5 to 7, wherein the composition is administered topically, orally or by means of an intraarticular, subcutaneous, intravenous or muscular injection.
